Europäisches Patentamt

European Patent Office

Office européen des brevets

(19)

(11) Numéro de publication: **0 245 641**
**B1**

(12) **FASCICULE DE BREVET EUROPEEN**

(45) Date de publication du fascicule du brevet: **23.01.91**

(21) Numéro de dépôt: **87104787.4**

(22) Date de dépôt: **01.04.87**

(51) Int. Cl.⁵: **A 23 G 9/30,** A 23 L 2/20,
A 23 L 3/16

(54) **Machine pour le traitement et/ou la conservation de boissons ou de melanges alimentaires liquides ou pateuses.**

(30) Priorité: **28.04.86 IT 2023686**

(43) Date de publication de la demande:
**19.11.87 Bulletin 87/47**

(45) Mention de la délivrance du brevet:
**23.01.91 Bulletin 91/04**

(84) Etats contractants désignés:
**AT BE CH DE ES FR GB GR LI NL SE**

(56) Documents cités:
**FR-A-1 021 060**
**US-A-4 372 916**

(73) Titulaire: **SIPP S.p.A.**
**Via Emilia**
**I-20070 Guardamiglio Milan (IT)**

(72) Inventeur: **Soffientini, Pierluigi**
**21, Viale Manzoni**
**I-20073 Codogno (Milan) (IT)**

(74) Mandataire: **Marietti, Giuseppe**
**CENTRO DI CONSULENZA IN PROPRIETA'**
**INDUSTRIALE Viale Caldara, 43**
**I-20122 Milano (IT)**

EP 0 245 641 B1

Courier Press, Leamington Spa, England.

## Description

La présente invention concerne les machines destinées au traitement et/ou à la conservation de boissons ou de mélanges alimentaires liquides ou pâteuses. Lesdites machines peuvent être utilisées dans les secteurs les plus différents et à titre d'exemple non limitatif on peut citer les distributeurs de comptoir pour boissons, du type "premix" et "postmix", les distributeurs pour la bière et le vin, les machines pour le traitement de mélanges glacées comme les pasteurisateurs, les cuves de maturation, les mélangeurs, les machines pour les glaces "express" ou semblables, les machines pour fouetter la crème chantilly, pour cuire les crèmes et en général toutes les machines qui sont spécialement utilisées dans le domaine artisanal et sont caractérisées en ce qu'elles disposent d'un robinet ou conduit de débit instantané, ayant une zone qui est intéressée per la substance en phase de distribution et d'autre part peut aussi se trouver en contact avec le milieu extérieur, substantiellement à la température de ce dernier, lorsque ladite distribution est suspendue.

Dans lesdites machines ou appareils il arrive que des restes de substance alimentaire demeurent dans ladite zone du robinet ou conduit après l'interruption de la distribution, et entrent ainsi en contact avec le milieu extérieur dans une situation telle à favoriser de développement de germes pathogènes, au moins après une période de temps déterminée.

C'est pourquoi il est très important pour la santé publique que ladite zone du robinet ou conduit de débit soit constamment maintenue dans des conditions stériles et que l'on intervienne sur la même chaque fois que la distribution de la substance alimentaire est suspendue ou que l'on prévoit que'elle soit suspendue pour une période de temps suffisante au développement de germes pathogènes, par exemple pour toute une nuit, l'intervention devant être effectuée avant que la distribution recommence.

Plusieurs propositions ont été avancées pour resoudre ledit problème, y compris le désassemblage et le lavage soigné des parties de machine intéressées, le maintien desdites pièces dans des conditions thermiques telles à empêcher le développement de germes pathogènes et l'intervention par des appareils de lavage et/ou de stérilisation exterieurs. Cependant toutes ces propositions n'ont pas rencontré un succès complet à cause des coûts, ou bien à cause du fait qu'elles entrainaient nécessairement pour l'opérateur une certaine masse de travail et une certaine perte de temps pour assurer le débit désiré dans des conditions stériles.

La présente invention se propose maintenant le but de réaliser une machine dudit type et pour lesdites utilisations, dans laquelle il est possible d'effecteur avec sécurité et précision, à commande, le lavage et la stérilisation du robinet ou conduit de débit, chaque fois que l'opérateur le juge nécessaire et en pratique sans aucun travail ou perte de temps pour ledit opérateur.

Selon l'invention, ce qui précède est obtenu, dans une machine comme définie auparavent, qui comprend, dans son enveloppe:

une prise pour prélever de l'eau du réseau ou d'une autre source;

une chaudière pour produire de la vapeur et éventuellement de l'eau chaude, alimentée avec l'eau de ladite prise;

des dispositifs de contrôle des conditions de production de la vapeur et éventuellement de l'eau chaude dans la chaudiére;

une conduite pour alimenter la vapeur produite au robinet ou au conduit de débit de la substance alimentaire, de sorte qu'elle intéresse toute la zone définie ci-dessus; et

un élément de commande pour donner lieu à un cycle temporisé de production et alimentation de la vapeur.

Comme on le verra mieux par la suite, dans une machine concue de cette sorte, il est possible pour l'opérateur d'effectuer un cycle de stérilisation toutes les fois qu'il le désire, simplement en agissant sur un poussoir. A la fin d'un délai pré-établi, en général de 1,5 à 5 minutes selon la substance traitée et le temps de permanence des restes dans la zone de débit susmentionnée, le cycle se termine et la machine se trouve automatiquement dans les conditions de distribuer encore la substance alimentaire traitée, sous des conditions hygiéniques parfaites.

L'invention sera maintenant décrite plus en détail avec référence aux dessins schématiques annéxes dans lequels:

—la figure 1 est une vue en perspective schématique d'une machine comprenant dans son enveloppe des moyens de stérilisation;

—la figure 2 est un schéma en blocs montrant le circuit hydraulique et les connections électriques desdits moyens de stérilisation;

—la figure 3 est un coupe transversale d'une forme de réalisation possible d'une petite chaudière productrice de vapeur;

—la figure 4 est une vue en plan de la partie inférieure de la petite chaudière de la figure 3, montrant le labyrinthe suivi par l'eau et la vapeur entre le point de captation de l'eau et le point de prise de la vapeur.

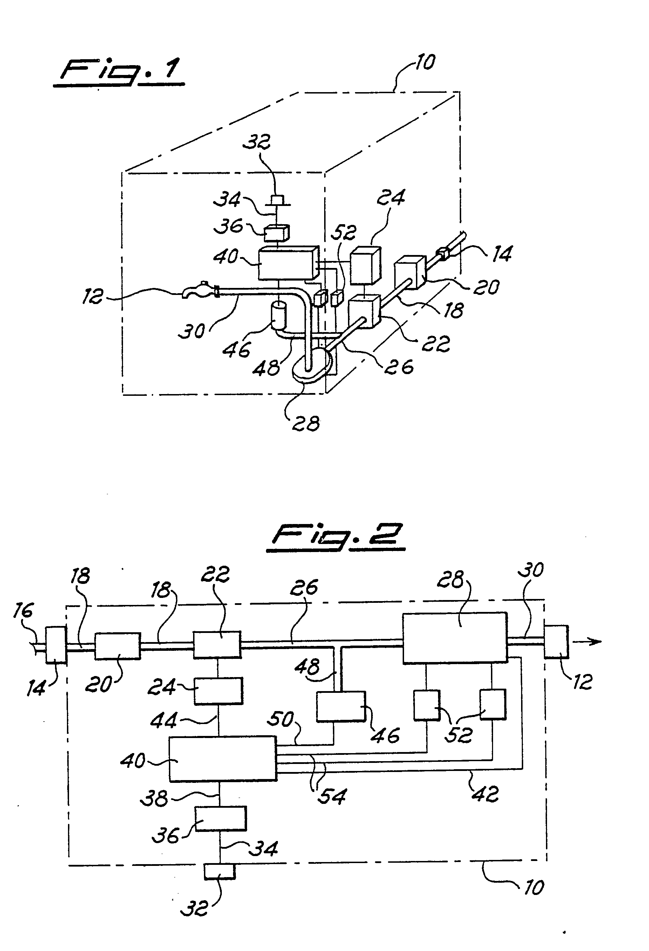

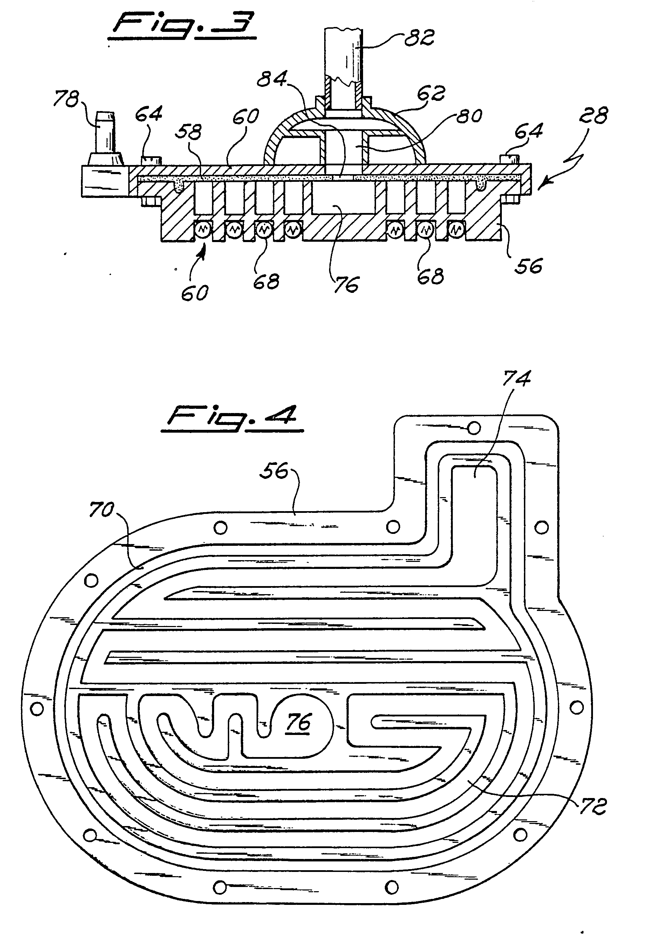

En considérant d'abord les figures 1 et 2, la référence 10 indique l'enveloppe d'une machine pour le traitement et/ou la conservation de substances alimentaires, par exemple un distributeur "postmix" de comptoir, lequel prévoit un robinet distributeur 12 qui récoit simultanément de l'eau gazéifiée froide et un ou plusieurs sirops pour la préparation instantanée d'une boisson. Ledit robinet présente une zone, au cas spécifique en aval du point de mélange, qui est léchée par la boisson distribuée, mais qui cependant reste en contact avec le milieu extérieur, substantiellement à la température de ce dernier, lorsque la distribution est suspendue. Au cas où ladite permanence se prolonge pendant quelques heures, il est nécessaire d'effectuer le nettoyage et la stérilisation de la zone précitée avant de recommencer la distribution. Ce problème, comme déjà dit, se présente dans un grand nombre de machines pour le traitement et/ou la conservation de substances alimentaires liquides ou pâteuses, auxquelles s'applique la présente invention.

Pour effectuer ladite stérilisation on prévoit une série de dispositifs logés dans l'enveloppe 10 et comprenant une prise 14 reliée en 16 au réseau d'alimentation ou à une autre source d'eau, ladite prise 14 alimentant l'eau, à travers une conduite 18 sur laquelle est monté un régulateur de débit 20, à une vanne 22 commandée électriquement en 24 pour contrôler l'envoi de l'eau dans une conduite 26 et depuis celle-ci à une petite chaudière 28 pour la production de vapeur. Cette dernière est pourvue d'une prise de vapeur qui alimente, à travers une conduite 30, le robinet ou conduit ditributeur 12, de sorte que toute la zone dangereuse du dernier soit intéressée par la vapeur, qui la lave et la stérilise, avant d'être déchargée dans l'atmosphère.

La phase de stérilisation est actionnée par l'opérateur par exemple an appuyant sur un poussoir 32, électriquement relié en 34 à un temporisateur 36 qui est ainsi activé pour commencer le cycle. Le temporisateur agit à son tour, grâce à une fiche électronique de contrôle 40 à laquelle il est relié en 38, d'abord sur les résistances électriques (pas illustrées) de la chaudière 28 (connexion 42) et ensuite, lorsque la petite chaudière 28 est à la température appropriée, sur l'électrovanne 22, 24 pour contrôler l'alimentation de l'eau à la chaudière 28 et ainsi les conditions de formation et d'envoi de la vapeur au robinet 12.

Lesdites conditions de formation de la vapeur sont avant tout contrôlées par des dispositifs de sécurité, comprenant un régulateur de pression 46 relié hydrauliquement au moyen d'un embranchement en T 48 à la conduite 26 et électriquement en 50 à la fiche électronique 40, qui coupe l'alimentation d'énergie électrique à la chaudière 28 si la vapeur atteint des pressions dangereuses. D'autres dispositifs de sécurité sont constitués par des détecteurs de température 52 placés dans des points appropriés de la chaudière 28 et reliés en 54 à la fiche électronique 40, qui coupe l'alimentation d'énergie électrique à la chaudière si l'on atteint des températures dangereuses.

Les réglages ne prévoient pas seulement le réglage du temporisateur 36 sur un temps de cycle approprié, en général de 1,5 à 5 minutes selon la substance distribuée, mais aussi un réglage des conditions d'alimentation de l'eau à la chaudière au moyen de l'électrovanne 22, 24. Celle-ci est commandée par la fiche électronique 40 de façon qu'elle s'ouvre et se ferme avec une fréquence déterminée et que ses temps d'ouverture soient contrôlés, afin de réaliser des conditions d'alimentation de l'eau à la chaudière et donc de formation et d'alimentation de la vapeur au robinet 12 qui soient les plus appropriées à l'application particulière. L'alimentation de l'eau à la chaudière 28 peut avoir aussi un débit tel à dépasser, éventuellement pendant un certain temps seulement, les capacités de vaporisation complète de la chaudière même, de façon que le robinet 12 reçoit ainsi un mélange de vapeur et d'eau chaude.

Les figures 3 et 4 môntrent une possible forme de réalisation de la chaudière 28 qui est ici essentiellement constituée par trois parties: un corps inférieur métallique 51, une garniture d'étanchéité 58 et un corps supérieur métallique de fermeture 60, comprenant un couvercle 62 pour recueillir et distribuer la vapeur, les deux corps 56 et 60 étant fixés entre eux par exemple par des vis 14. Le corps inférieur 56 présente extérieurement des sièges 66 pour le logement des résistances 68 de chauffage, tandis que sa partie supérieure est pourvue d'une série de nervures qui définissent (figure 4) un siège 70 pour le logement d'un bord correspondant de la garniture 58, ainsi qu'un labyrinthe fermé dans la partie supérieure par la garniture 58. Ce labyrinthe doit être nécessairement parcouru par l'eau et la vapeur à partir d'une cuve 74 à proximité de la prise 78 de captation de l'eau, jusqu'à un petit puits 76 qui se trouve en correspondance d'ouvertures 84, dans la garniture 58, et 80 dans le corps supérieur 60, pour alimenter la vapeur au couvercle 62 et au raccord 82 d'envoi de la vapeur à la conduite 30. Cette configuration de la petite chaudière 28 permet d'atteindre rapidement les conditions opératives et donc d'effectuer tout le cycle de stérilisation avec une consommation d'énergie très réduite. D'autre part, comme on le voit d'après la description qui précède, l'invention permet d'effectuer à discrétion des cycles de stérilisation avec la confiabilité le meilleure et sûrtout sans aucun travail et perte de temps pour l'opérateur, qui doit se borner à actionner un poussoir.

**Revendications**

1. Machine pour le traitement et/ou la conservation de boissons ou mélanges alimentaires liquides ou pâteuses, ayant un robinet ou conduit pour la distribution instantanée, une zone duquel est intéressée par la substance en phase de distribution et se trouve en contact avec le milieu extérieur lorsque cette distribution est suspendue, caractérisée en ce qu'elle comprend, dans son enveloppe:

une prise pour prélever de l'eau du réseau ou d'une autre source;

une chaudière pour produire de la vapeur et éventuellement de l'eau chaude, alimentée avec l'eau de ladite prise;

des dispositifs de contrôle des conditions de production de la vapeur et éventuellement de l'eau chaude dans la chaudière;

une conduite pour alimenter la vapeur produite au robinet ou au conduit de débit de la substance alimentaire, de sorte qu'elle intéresse toute la zone définie ci-dessus; et

en élément de commande pour donner lieu à un cycle temporisé de production et alimentation de la vapeur.

2. Machine selon la revendication 1, caractérisée en ce que les dispositifs de contrôle des conditions de production de la vapeur comprennent une électrovanne qui commande l'alimentation de l'eau à la chaudière au moyen de cycles d'ouverture et de fermeture réglables à discrétion quant à fréquence et temps d'ouverture.

3. Machine selon la revendication 2, caractérisée par le fait de comprendre, entre la prise d'alimentation et l'électrovanne, un régulateur de débit.

4. Machine selon la revendication 2 ou 3, caractérisée en ce que l'élément de commande comprend un poussoir, l'actionnement duquel commence une série de cycles d'ouverture et de fermeture de l'électrovanne, ainsi que l'activation d'un temporisateur qui contrôle et termine ladite série de cycles.

5. Machine selon la revendication 1, caractérisée en ce que la chaudière est pourvue de moyens de contrôle de sécurité des conditions de fonctionnement, comprenant au moins un détecteur de pression et au moins un détecteur de température capables d'arrêter le chauffage si l'on atteint des conditions de pressione et/ou de température dangereuses.

6. Machine selon la revendication 1, caractérisée en ce que la chaudière comprend une plaque qui peut être chauffée et qui présente des saillies formant, en coopération avec le couvercle de fermeture à étanchéité, un labyrinthe parcouru obligatoirement par l'eau et la vapeur entre le point d'entrée de l'eau et le point de prise de la vapeur et eventuellement de l'eau chaude.

**Patentansprüche**

1. Vorrichtung für die Behandlung und/oder Konservierung von Getränken oder flüssigen oder pastösen Nahrungsmittelmischungen mit einem Hahn oder einer Leitung zur augenblicklichen Verteilung, wovon eine Zone von der Substanz in der Phase der Verteilung bestrichen ist und sich in Kontakt mit der äßßeren Umgebung befindet, sobald die Verteilung beendet ist, dadurch gekennzeichnet, daß sie in ihrem Gehäuse umfaßt:
einen Anschluß zur Entnahme von Wasser aus dem Leitungsnetz oder einer anderen Quelle;
einen vom Wasseranschluß gespeisten Kessel zur Erzeugung von Dampf und gegebenenfalls von Heißwasser;
eine Einrichtung zur Steurung der Herstellungsbedingungen für den Dampf und gegebenenfalls für das Heißwasser im Kessel; eine Leitung zur Zuführung des hergestellten Dampfes zum Auslaßhahn oder zur Auslaßleitung für die Nahrungsmittelmasse in der Weise, daß sie die gesamte oben definierte Zone bestreicht;
und ein Steuerelement, das eine Verzögerungsschaltung für die Herstellung und Zufuhr von Dampf steuert.

2. Vorrichtung nach Anspruch 1, dadurch gekennzeichnet, daß die Einrichtungen zur Steuerung der Bedingungen der Herstellung von Dampf ein Elektroventil umfassen, das die Zufuhr von Wasser zum Kessel über beliebig hinsichtlich Frequenz und Dauer des Öffnens regelbare Öffnungs- und Schließzyklen steuert.

3. Vorrichtung nach Anspruch 2, dadurch gekennzeichnet, daß zwischen dem Zufuhrleitungsanschluß und dem Elektroventil ein Auslaßregler vorgesehen ist.

4. Vorrichtung nach Anspruch 2 oder 3, dadurch gekennzeichnet, daß das Steuerelement einen Druckschalter umfaßt, durch dessen Betätigung eine Reihe von Öffnungs- und Schließzyklen des Elektroventils beginnt ebenso wie die Aktivierung eines Verzögerers, der die Reihe von Zyklen steuert und beendet.

5. Vorrichtung nach Anspruch 1, dadurch gekennzeichnet, daß der Kessel mit Sicherheitssteuerorganen für die Funktionsbedingungen versehen ist, die mindestens einen Druckfühler und mindestens einen Temperaturfühler zum Stoppen der Heizung umfaßt, wenn gefährliche Druck- und/oder Temperaturbedingungen erreicht werden.

6. Vorrichtung nach Anspruch 1, dadurch gekennzeichnet, daß der Kessel eine Platte umfaßt, die heizbar ist und Vorsprünge aufweist, die zusammen mit dem abdichtenden Verschlußdeckel ein Labyrinth bilden, das vom Wasser und dem Dampf zwischen Einlaßstelle des Wassers und Anschlußstelle für Dampf und gegebenenfalls warmes Wasser durchströmt wird.

**Claims**

1. A machine for treating and/or preserving drinks or liquid or pasty foodstuff mixtures having a tap or conduit for instantaneous distribution, a zone of which tap or conduit is involved with the substance in the distribution phase and is in contact with the external medium when said distribution is terminated, characterised in that it comprises, in its housing:
an intake for taking water from the mains system or another source;
a boiler for producing steam and possibly hot water, which is supplied with the water from said intake;
devices for monitoring the conditions for production of the steam and possibly hot water in the boiler;
a conduit for supplying the steam produced to the tap or to the flow conduit for the foodstuff substance, in such a way that it involves all the above-defined zone; and
a control element for giving rise to a timed steam production and supply cycle.

2. A machine according to claim 1, characterised in that the devices for monitoring the conditions for production of the steam comprise an electrically operated valve which controls the supply of water to the boiler by means of opening and closing cycles which can be regulated as desired in regard to the frequency and time of opening.

3. A machine according to claim 2, characterised by comprising a flow rate regulator between the supply intake and the electrically operated valve.

4. A machine according to claim 2 or claim 3, characterised in that the control element comprises a push button, the actuation of which begins a series of cycles of opening and closing of the electrically operated valve, as well as activation of a timer which monitors and terminates said series of cycles.

5. A machine according to claim 1, characterised in that the boiler is provided with safety means for monitoring the operating conditions comprising at least one pressure detector and at least one temperature detector which are capable of stopping the heating effect if dangerous conditions in respect of pressure and/or temperature are attained.

6. A machine according to claim 1, characterised in that the boiler comprises a plate which can be heated and which has projecting portions which, co-operating with the sealing closure cover, form a labyrinth through which the water and the steam necessarily pass between the water inlet point and the point of intake of the steam and possibly hot water.

_Fig.1_

_Fig.2_

_Fig.3_

_Fig.4_